# EUROPÄISCHE PATENTSCHRIFT

(11) **EP 2 340 431 B1**
(45) Veröffentlichungstag und Bekanntmachung des Hinweises auf die Patenterteilung: **06.09.2017**
(21) Anmeldenummer: 09783480.8
(22) Anmeldetag: 28.09.2009
(51) Int. Cl.: G01N 27/28, G01N 27/403, G01N 33/18

(54) **MOBILE WASSER-ANALYSEANORDNUNG UND VERFAHREN ZUR BESTIMMUNG EINES ANALYTS IN EINER WASSERPROBE**
MOBILE WATER ANALYSIS ARRANGEMENT AND METHOD FOR DETERMINING AN ANALYTE IN A WATER SAMPLE
DISPOSITIF MOBILE D'ANALYSE D'EAU ET PROCÉDÉ DE DÉTERMINATION D'UN ANALYTE DANS UN ÉCHANTILLON D'EAU

(30) Priorität: 06.10.2008 DE 102008050092
(43) Veröffentlichungstag der Anmeldung: 06.07.2011
(73) Patentinhaber: Hach Lange GmbH, 14163 Berlin (DE)
(72) Erfinder: LUNDGREEN, Ulrich, 33330 Gütersloh (DE); FARJAM, Aria, 40223 Düsseldorf (DE); UTHEMANN, Rolf, 51373 Leverkusen (DE); MITREITER, Andreas, 14532 Kleinmachnow (DE); HÜNIG, Isabel, 40547 Düsseldorf (DE); LENHARD, Markus, 41751 Viersen (DE); FRÖMEL, Rainer, 53842 Troisdorf (DE); KUMPCH, Hans-Joachim, 10999 Berlin (DE)
(74) Vertreter: Patentanwälte ter Smitten Eberlein-Van Hoof Rütten Partnerschaftsgesellschaft mbB
(86) Internationale Anmeldenummer: PCT/EP2009/062521
(87) Internationale Veröffentlichungsnummer: WO 2010/040655

(56) Entgegenhaltungen:
- EP-A1- 1 870 033
- EP-A2- 0 821 231
- WO-A2-02/074043
- DE-A1- 10 126 054
- US-A- 5 731 212
- US-A- 5 821 405
- US-A1- 2004 154 933
- US-A1- 2004 241 051
- US-A1- 2007 144 277
- US-A1- 2008 227 185

## Beschreibung

Die Erfindung bezieht sich auf eine mobile Wasser-Analyseanordnung zur Bestimmung eines Analyts in einer Wasserprobe. Unter "mobil" wird vorliegend eine Analyseanordnung verstanden, die nicht stationär verbaut ist, wie dies beispielsweise bei quasi-kontinuierlich messenden Prozess-Analysegeräten der Fall ist.

Im Bereich der mobilen Wasseranalytik stellen gegenwärtig u.a. sogenannte fotometrische Küvettentests den Stand der Technik dar, wie er beispielsweise in DE 41 09 118 A1 beschrieben ist. Die Durchführung des Küvettentests erfolgt praktisch rein manuell. Zunächst wird mit einer Pipette eine Wasserprobe aufgenommen. Die auf diese Weise gewonnene Wasserprobe wird in eine Küvette gegeben, die ein Hauptreagenz enthält. Die Küvette wird verschlossen und zum Vermischen der Wasserprobe und des Hauptreagenzes geschüttelt. Schließlich wird die Küvette in ein Fotometer eingesetzt und fotometriert.

Die Durchführung des manuellen Küvettentests ist umständlich und äußerst fehleranfällig. Die verwendete Hauptreagenz kann umwelt- oder gesundheitsschädlich sein. Gegebenenfalls muss daher die Küvette nach Durchführung der Analyse entsprechend entsorgt werden. Wegen der umständlichen Handhabung können die Küvettentests praktisch nur im Labor durchgeführt werden.

Aus US 2007/0144277 A1 ist ein mobiles Blut- und Urin-Analysegerät bekannt, das eine Basiseinheit und eine Wegwerf-Kartusche aufweist. Die Wegwerf-Kartusche weist zur Probenaufnahme ein Septum auf, durch das hindurch die Probe mit Hilfe einer Spritze in die Kartusche appliziert werden muss.

Aus EP 0821231 A2, WO 02074043 A2 und DE 10126054 A1 sind modular aufgebaute Prozess-Analysegeräte bekannt, die jeweils ein Basisgerät mit der Gerätesteuerung und eine austauschbare Kartusche mit der Probenaufnahme und Reagenzientanks aufweisen.

Aus US 5731212 A ist ein Testelement bekannt, bei dem die Messgeräte quer zur Probenleitung angeordnet sind und messen.

Aufgabe der Erfindung ist es, eine mobile Wasser-Analyseanordnung und ein Verfahren zur Bestimmung eines Analyts einer Wasserprobe mit vereinfachter Handhabung zu schaffen.

Diese Aufgabe wird erfindungsgemäß durch eine Wasser-Analyseanordnung mit den Merkmalen des Patentanspruches 1 beziehungsweise durch ein Verfahren zur Bestimmung eines Analyts in einer Wasserprobe mit den Merkmalen des Patentanspruches 11 gelöst.

Das Hauptreagenz kann zwischen der Einlassöffnung und dem Messabschnitt angeordnet sein, kann jedoch auch zwischen dem Messabschnitt und der Pumpöffnung angeordnet sein.

Die Basiseinheit weist eine Testelement-Aufnahme zum Halten des eingesteckten Testelementes auf. Ferner weist die Basiseinheit ein Messgerät auf, mit dem die Wasserprobe in dem Messabschnitt fotometrisch oder elektrochemisch vermessen wird. Schließlich weist die Basiseinheit einen Pumpenaktuator auf, der mit der Pumpöffnung des eingesteckten Testelementes zusammenwirkend verbunden ist und der für das Ansaugen einer Wasserprobe in die Probenleitung und für den Transport der Wasserprobe durch die Probenleitung sorgt.

Die Bestimmung eines Analyts einer Wasserprobe erfolgt, indem zunächst ein Testelement manuell oder automatisch in die Basiseinheit eingesteckt beziehungsweise appliziert wird. Anschließend wird die Einlassöffnung manuell in das zu untersuchende Wasser eingetaucht und der Pumpenaktuator eingeschaltet. Das Einschalten des Pumpenaktuators kann manuell, kann jedoch auch automatisch erfolgen. Durch das Einschalten des Pumpenaktuators wird eine Wasserprobe durch die Einlassöffnung der Probenleitung aktiv in Richtung Messabschnitt gepumpt.

Es wird eine definierte Menge der Wasserprobe in Form einer Probensäule angesaugt und isoliert, die an beiden Enden durch Luft begrenzt ist. Durch die Beschränkung auf eine Probensäule definierten Volumens wird ein definiertes Verhältnis zwischen der Wasserprobe und dem Hauptreagenz sichergestellt. Ferner kann eine homogene Durchmischung des Hauptreagenz mit der Wasserprobe erreicht werden, indem die isolierte und mit dem Hauptreagenz beaufschlagte Wasserproben-Säule in der Probenleitung mehrmals hin und her gepumpt wird, wodurch sich in der Wasserproben-Säule auf Grund der Reibung an der Leitungswand Strömungen und Turbulenzen ergeben.

Die Begrenzung der Wasserproben-Menge auf eine definierte Menge kann beispielsweise dadurch erfolgen, dass nach dem Ansaugen der definierten Wasserproben-Menge der Pumpenaktuator anhält und ein Probennahme-Endsignal ausgibt, das den Bediener auffordert, die Einlassöffnung aus dem zu untersuchenden Wasser wieder heraus zu nehmen. Alternativ kann die Isolation der definierten Wasserproben-Menge auch automatisch über ein entsprechendes Ventil erfolgen, das nach dem Ansaugen der definierten Wasserproben-Säule Luft in die Probenleitung leitet.

Wenn das Hauptreagenz, von der Einlassöffnung aus gesehen, vor dem Messabschnitt angeordnet ist, wird die Wasserprobe auf den Weg von der Einlassöffnung zu dem Messabschnitt mit dem Hauptreagenz gemischt.

Wenn das Hauptreagenz zwischen dem Messabschnitt und der Pumpöffnung angeordnet ist, kann die Wasserprobe zunächst zur Messstrecke gepumpt werden, um dort mit Hilfe des Messgerätes einen Probenblindwert zu ermitteln, und um anschließend zu dem Hauptreagenz hinter dem Messabschnitt weiter gepumpt zu werden. Dort reagiert die Wasserprobe mit dem Hauptreagenz und wird anschließend wieder rückwärts in dem Messabschnitt gepumpt, um die quantitative Bestimmung des Analyts in der Wasserprobe vorzunehmen.

Auf dem Weg zu dem Messabschnitt wird das Hauptreagenz in der Probenleitung mit der Wasserprobe vermischt. Dies kann beispielsweise dadurch sichergestellt werden, dass zwischen dem Ort der Hauptreagenz-Einleitung und dem Messabschnitt ein bevorzugt relativ langer Mischabschnitt der Probenleitung liegt. Die homogene Vermischung kann zusätzlich beispielsweise durch richtungswechselnde Pumposzillationen intensiviert werden.

Das Hauptreagenz geht mit dem nachzuweisenden Analyt in der Wasserprobe eine Reaktion ein, die die optischen oder elektrochemischen Eigenschaften der Wasserprobe verändert. Bezogen auf das Beispiel eines als Fotometer ausgebildeten Messgerätes wird insbesondere das Absorptionsspektrum der Wasserprobe auf bestimmten Spektrallinien beziehungsweise in bestimmten Spektralbereichen verändert. Die auf diese Weise aufbereitete und homogenisierte Wasserprobe gelangt schließlich in den Messabschnitt, in dem sie durch das Messgerät der Basiseinheit elektrisch oder optisch vermessen wird.

Das Ergebnis der Messung wird aufbereitet, und gegebenenfalls angezeigt und/oder abgespeichert. Sobald das Messergebnis vorliegt, kann das Einmal-Testelement manuell oder automatisch entnommen beziehungsweise entfernt werden.

Das Testelement kann beispielsweise die Größenordnung eines breiten Streichholzes aufweisen. Die Probenleitung kann daher einen entsprechend kleinen Querschnitt haben, der im Bereich von 0,01 mm² bis zu mehreren Quadratmillimetern liegen kann. Der Fotometerabschnitt, beziehungsweise die von ihm gebildete Messstrecke, sollte möglichst lang sein, beispielsweise mehrere Millimeter bis zu mehreren Zentimetern. Das Volumen der Wasserprobe in dem Fotometerabschnitt liegt also im Bereich von einem bis zu circa fünfzig Kubikmillimetern. Entsprechend gering ist die hierfür erforderliche Hauptreagenz-Menge. Damit ist auch die durch das Hauptreagenz begründete Gefährdung für die Umwelt beziehungsweise die Gesundheit entsprechend gering. Hierdurch kann die Notwendigkeit einer geregelten Entsorgung entfallen, so dass ein erheblicher Aufwand für die geregelte Entsorgung beziehungsweise Rückführung des Testefementes zum Lieferanten beziehungsweise zum Hersteller entfällt.

Alle für die Qualität des Messergebnisses relevanten Arbeitsschritte der Wasseranalyse, beispielsweise die Dosierung des Hauptreagenz, die Vermischung des Hauptreagenz mit der Wasserprobe, das Abwarten der erforderlichen Reaktionszeit etc., erfolgen halbautomatisch beziehungsweise automatisch und verschlossen. Damit sind aus einer fehlerhaften Handhabung resultierende Fehler und Gefährdungen nahezu ausgeschlossen.

Die Messstrecke des Messabschnittes wird von einem Längsabschnitt der Probenleitung gebildet, das heißt, die Messstrecke verläuft in Längsrichtung der Probenleitung, nicht in Querrichtung. Hierdurch ist die Messstrecke erheblich länger, als wenn diese in Querrichtung der Probenleitung verlaufen würde. Je länger eine Messstrecke ist, desto genauer kann gemessen werden.

Das Messgerät ist als Fotometer mit einer Lichtquelle zur Erzeugung eines Messstrahles und einem Lichtempfänger zum Empfangen des Lichtstrahles, nachdem dieser den einen Fotometerabschnitt bildenden Messabschnitt des Testelementes durchlaufen hat, ausgebildet. Besonders bevorzugt ist das Fotometer als Transmissions-Fotometer ausgebildet. Ein Transmissions-Fotometer hat im Vergleich zum Reflektions-Fotometer ein relativ großes Nutzsignal. Dies ermöglicht auch bei einer relativ kurzen Messstrecke eine präzise quantitative Ermittlung des Analyts in der Wasserprobe. Der Messabschnitt weist mindestens ein Fotometer-Fenster für den Eintritt und den Austritt eines Fotometer-Messstrahles auf. Fotometrisch können beispielsweise Chlor, Phosphate und Ammonium bestimmt werden.

Gemäß einer bevorzugten Ausgestaltung wird das Einmal-Testelement im Wesentlichen aus einem Bodenteil und einem separaten Deckelteil gebildet, die zwischen sich die Probenleitung bilden. Beispielsweise kann das Bodenteil ein Kunststoff-Spritzgussteil sein, in dem eine U-förmige offene Nut gebildet ist. Das Deckelteil kann als transparente Kunststoff-Folie ausgebildet sein, die auf die Nutseite des Bodenteiles aufgeschweißt oder geklebt ist, nachdem das Hauptreagenz und gegebenenfalls die Hilfsreagenzien und Hilfssubstanzen in die Probenleitung eingebracht wurden. Nur durch die zweiteilige Ausbildung des Testelementes ist es möglich, die Reagenzien und Substanzen genau platziert, in die Probenleitung einzubringen. Dies ist insbesondere auch deshalb so, weil der Querschnitt der Probenleitung maximal mehrere Quadratmillimeter beträgt.

Alternativ kann das Messgerät auch die Trübung beziehungsweise Streuung messen.

Die Basiselnheit kann sowohl ein fotometrisches beziehungsweise optisches als auch ein elektrochemisches Messgerät aufweisen, so dass sowohl ein optisches beziehungsweise fotometrisches als auch ein elektrochemisches Testelement in der Basiseinheit alternativ betrieben werden können.

Das Testelement kann mindestens ein Positionierelement aufweisen, das eine exakte Positionierung des Testelementes in der Basiseinheit gewährleistet. Um insbesondere eine fehlerfreie Fotometrie sicherstellen zu können, muss der Messabschnitt des Testelementes exakt mit dem Fotometer der Basiseinheit ausgerichtet sein. Hierzu ist mindestens ein separates Positionierelement vorgesehen, das neben den Seitenflächen des Testelementes für eine zusätzliche exakte Positionierung des Testelementes in der Basiseinheit sorgt. Beispielsweise kann in dem Testelement eine Nut, eine Vertiefung oder eine durchgehende Öffnung vorgesehen sein, in die ein entsprechendes vorgespanntes Rastelement der Basiseinheit eingreift, um das Testelement in der Basiseinheit exakt zu positionieren und zu fixieren.

Das Hauptreagenz ist, beispielsweise in getrockneter Form, im Verlauf der Probenleitung angeordnet.

Gemäß einer bevorzugten Ausgestaltung ist in der Probenleitung zusätzlich zu dem Hauptreagenz ein Hilfsreagenz vorgesehen. Das Hilfsreagenz kann, abhängig von seiner Funktion, zwischen der Einlassöffnung und den Messabschnitt oder zwischen dem Messabschnitt und der Pumpöffnung angeordnet sein.

Das Hilfsreagenz kann beispielsweise ein Aktivator sein, der das Hauptreagenz aktiviert, sobald es mit dem Hilfsreagenz in der Wasserprobe vermischt wird. Auf diese Weise kann die Haltbarkeit, die Ungefährlichkeit und/oder die Unempfindlichkeit des Hauptreagenzes gegebenenfalls verbessert werden.

Das Hilfsreagenz kann ein separates Reagenz sein, das zum Nachweis eines zweiten Analyts in der Wasserprobe dient. Das Hilfsreagenz kann, aufbauend auf der bzw. nach der Reaktion des Hauptreagenzes mit der Wasserprobe eine mehrstufige Reaktion bewirken. Das Hilfsreagenz kann auch ein Analyt-Standard sein, mit dessen Hilfe eine Standardaddition durchgeführt werden kann.

Das Hilfsreagenz kann auch ein Neutralisationsreagenz sein, das zwischen dem Messabschnitt und der Pumpöffnung angeordnet ist. Nach der Bestimmung des Analyts in dem Messabschnitt wird die Wasserprobe zu dem Hilfsreagenz gepumpt, das mit dem Hauptreagenz derart reagiert, dass das Hauptreagenz neutralisiert wird.

Das Hilfsreagenz kann auch gelierend und/oder verfärbend in der Wasserprobe wirken, und nach Durchführung der Bestimmung des Analyts in den Messabschnitt mit der Wasserprobe zusammengeführt werden. Durch die Verfärbung wird deutlich sichtbar gemacht, dass das Testelement bereits benutzt wurde. Durch die Gelierung wird die Wasserprobe in der Probenleitung fixiert, und kann nicht mehr auslaufen.

Selbstverständlich können auch mehrere verschiedene Reagenzien hintereinander in der Probenleitung vorgesehen sein, die der Bestimmung verschiedener Analyte in derselben Wasserprobe dienen. Sobald die Wasserprobe das betreffende Reagenz erreicht, löst sich dieses in der Wasserprobe und reagiert mit dem zu bestimmenden Analyt beispielsweise farbverändernd. Das Analyt wird in dem Messabschnitt bestimmt, und die Wasserprobe anschließend dem nächsten Reagenz zur Bestimmung eines zweiten Analyts in der Wasserprobe zugeführt.

Vorzugsweise weist das Testelement eine die Pumpöffnung flüssigkeitsdicht und gasdicht verschließende Pumpenmembran auf, die ein Pumpenvolumen einschließt, das größer ist als das gesamte Pumpleitungs-Volumen. Dadurch ist es möglich, dass mit einem einzigen Hub der Pumpenmembran die Wasserprobe vorwärts beziehungsweise rückwärts über die gesamte Länge der Probenleitung bewegt und genau platziert werden kann. Die Pumpenmembran wird von einem basiseinheitseitigen Pumpenaktuator betätigt, der unter anderem einen entsprechenden Pumpenstößel aufweist, der wiederum die Pumpenmembran deformiert. Beispielsweise wird durch Eindrücken der Pumpenmembran durch den Pumpenstößel die Wasserprobe rückwärts in Richtung Einlassöffnung gepumpt, und durch Losfassen oder Ziehen der Pumpemembran durch eine Rückzug-Bewegung des Pumpenstößels die Wasserprobe vorwärts in Richtung Pumpöffnung gepumpt.

Gemäß einer alternativen Ausgestaltung ist der Pumpenaktuator ein Teil einer basiseinheitseitigen Probenpumpe, wobei die Probenpumpe mit der Pumpöffnung des eingesteckten Testelementes verbunden ist. Alle beweglichen Teile der Pumpe sind also an der Basiseinheit angeordnet. Die Basiseinheit und/oder das Testelement können im Bereich der Pumpöffnung ein elastisches Dichtelement aufweisen, das eine leckfreie Verbindung zwischen der Pumpöffnung und der Probenpumpe herstellt. Eine gasdichte und flüssigkeitsdichte Verbindung zwischen der Pumpöffnung und der Probenpumpe ist Voraussetzung für einen dauerhaft zuverlässigen und fehlerfreien Betrieb. Durch das elastische Dichtelement wird gewährleistet, dass die Probenpumpe bei jedem Testelement in leckdichter fluidischer Verbindung mit der Probenleitung steht. Nur auf diese Weise ist auch über große Dauer und auch noch nach vielen Messungen ein zuverlässiger Analysebetrieb sichergestellt.

Das Testelement kann als Multi-Analyt-Testelement ausgebildet sein und mindestens zwei separate Probenleitungen mit jeweils verschiedenen Hauptreagenzien aufweisen. Mit einem einzigen Testelement können auf diese Weise zwei oder mehr verschiedene Analyte bestimmt werden. Für jedes Analyt ist eine komplette Analyse-Mimik auf dem Testelement angeordnet, jeweils bestehend aus der Probenleitung mit Einlassöffnung und Pumpöffnung, einem Messabschnitt sowie einem entsprechenden Hauptreagenz. Mit einem einzigen Messzyklus können auf diese Weise mehrere Analyte bestimmt werden.

Das Testelement kann streifenförmig ausgebildet sein. Das streifenförmige Testelement kann beispielsweise aus einem flachen langgestreckten Spritzgussteil bestehen, in dem die Probenleitung einschließlich des Messabschnittes in Form einer Nut ausgebildet ist, in die das Hauptreagenz in den Verlauf der Probenleitung eingefüllt ist und das schließlich durch eine Folie verschlossen wurde.

Das beziehungsweise die Fotometer-Fenster des Fotometerabschnittes können als separate transparente Fenster ausgebildet sein. Alternativ kann das lang gestreckte Spritzgussteil vollständig aus einem transparenten Kunststoff bestehen.

Es können mehrere Testelemente in einer austauschbaren Kassette angeordnet sein, die in die Basiseinheit eingesetzt beziehungsweise einsetzbar ist. Die Kassette kann beispielsweise trommelförmig ausgebildet sein, wobei in den Trommelkammern jeweils ein Testelement hermetisch versiegelt bevorratet ist. Mit der austauschbaren Kassette und einem entsprechenden manuellen, halbautomatischen oder automatischen Applizierungs- und Auswurf- Mechanismus können mehrere Testelemente nacheinander verwendet werden, ohne dass diese rein manuell appliziert oder entnommen werden müssen.

Gemäß einer bevorzugten Ausgestaltung ist ein Absorptionskörper zwischen dem Messabschnitt und der Pumpöffnung angeordnet. Der Absorptionskörper dient der Aufnahme der Wasserprobe, nachdem diese im Messabschnitt analysiert und vorwärts zu dem Absorptionskörper gefördert wurde. Hierdurch wird die Wasserprobe immobilisiert, und ein Auslaufen der Wasserprobe aus dem Testelement heraus verhindert. Der Absorptionskörper kann beispielsweise ein Vlieskörper, ein Tonkörper beispielsweise aus Bentonit, oder ein so genannter Superabsorber sein. Der Absorptionskörper kann zusätzlich ein Neutralisationsreagenz enthalten.

Vorzugsweise ist in der Probenleitung ein Probenfilter angeordnet, der die durch die Einlassöffnung angesaugte Wasserprobe filtert, bevor diese zu dem Messabschnitt befördert und dort insbesondere fotometrisch analysiert wird. Der Probenfifter kann beispielsweise aus Glaswolle bestehen.

Gemäß einer bevorzugten Ausgestaltung weist die Basiseinheit ein Heiz- und/oder Kühlelement zum Heizen beziehungsweise zum Kühlen des Testelementes auf. Das Heizen beziehungsweise Kühlen kann geregelt sein, so dass eine konstante Temperatur des Testelementes eingestellt wird. Das Heizen des Testelementes auf beispielsweise 40°C kann dazu dienen, die Reaktion zwischen dem Analyt in der Wasserprobe und dem Reagenz erheblich zu beschleunigen beziehungsweise die Reaktion zu stabilisieren. Das Kühlen des Testelementes beziehungsweise der Wasserprobe in der Probenleitung kann insbesondere die Ausgasung, also die Bildung von für die Fotometrie sehr störende Gasblasen verhindern.

Vorzugsweise ist in der Probenleitung eine hydrophobe Stopperkapillare angeordnet. Die Stopperkapillare kann in der Nähe der Pumpöffnung angeordnet sein, so dass verhindert wird, dass die Wasserprobe die Probenleitung verlässt und durch die Pumpöffnung in die Basiseinheit gelangt.

Gemäß einer bevorzugten Ausgestaltung ist in der Probenleitung zwischen der Einlassöffnung und dem Messabschnitt eine Dosierkapillare angeordnet. Bevorzugt ist die Dosierkapillare unmittelbar an der Einlassöffnung vorgesehen. Durch die Dosierkapillare wird bei der Wasserprobennahme die Wasserprobe im wesentlichen durch die Kapillarkraft der Dosierkapillare gefördert, und auf diese Weise eine definierte Wasserprobe-Menge isoliert. Erst anschließend hieran wird die Wasserprobe durch den Pumpenaktuator aktiv in der Probenleitung vorwärts beziehungsweise gegebenenfalls rückwärts gefördert.

Das Reagenz kann in unmittelbarer Nähe zur Einlassöffnung angeordnet sein, um eine Interaktion von unreagiertem Analyt mit der Wand der Probenleitung zu vermeiden beziehungsweise zu minimieren. Dies ist beispielsweise bei der Bestimmung von freiem Chlor sinnvoll, wenn das Testelement beziehungsweise die Wand der Probenleitung aus Kunststoff besteht.

An dem Testelement kann ein Trockenmittel angeordnet sein, um das Hauptreagenz vor Feuchtigkeit zu schützen. Das Trockenmittel kann beispielsweise durch eine hydrophobe Stopperkapillare von der Probenleitung getrennt sein, durch die hindurch Feuchtigkeit aus der Probenleitung zu dem Trockenmittel gelangen kann.

Die Einlassöffnung und/oder die Pumpöffnung können mit einer feuchtigkeitsdichten Transportversiegelung versiegelt sein, die beim Einstecken des Testelementes in die Basiseinheit automatisch und oder manuell geöffnet, beispielsweise durchgestochen wird. Alternativ oder ergänzend kann das Testelement einzeln in eine feuchtigkeitsdichte Verpackung eingeschweißt sein.

Im Folgenden werden unter Bezugnahme auf die Zeichnungen mehrere Ausführungsbeispiele der Erfindung näher erläutert.

Es zeigen:
- Figur 1: eine schematische Darstellung einer mobilen Wasser-Analyseanordnung, bestehend aus einer Basiseinheit und einem Testelement,
- Figur 2: das Testelement der Analyseanordnung der Figur 1,
- Figur 3: ein zweites Ausführungsbeispiel einer mobilen Wasser-Analyseanordnung einschließlich einer austauschbaren Kassette mit mehreren Testelementen,

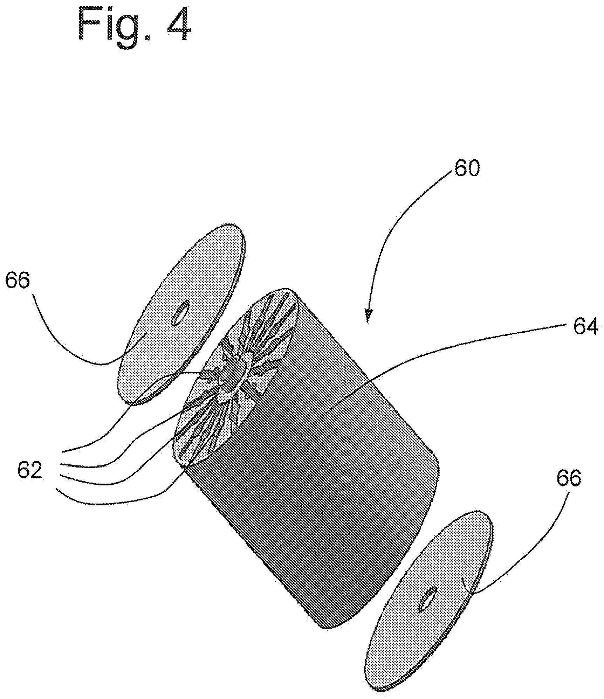

- Figur 4: die austauschbare Kassette der Figur 3,
- Figur 5: ein Beispiel eines nicht erfindungsgemässen elektrochemischen Testelementes in Vorderansicht,
- Figur 6: eine Rückansicht des Testelementes der Figur 5,
- Figur 7: eine Seitenansicht einer weiteren Ausführungsform eines Testelementes mit einer Pumpenmembran, die von einem Pumpenaktuator der Basiseinheit betätigt wird,
- Figur 8: eine weitere Ausführungsform eines Testelementes mit einem Hauptreagenz und einem Hilfsreagenz zwischen der Einlassöffnung und dem Messabschnitt,
- Figur 9: eine weitere Ausführungsform eines Testelementes mit einem Hauptreagenz und einem Hilfsreagenz zwischen dem Messabschnitt und der Pumpöffnung, und
- Figur 10: das Testelement der Figur 9 im Querschnitt entlang der Linie X-X.

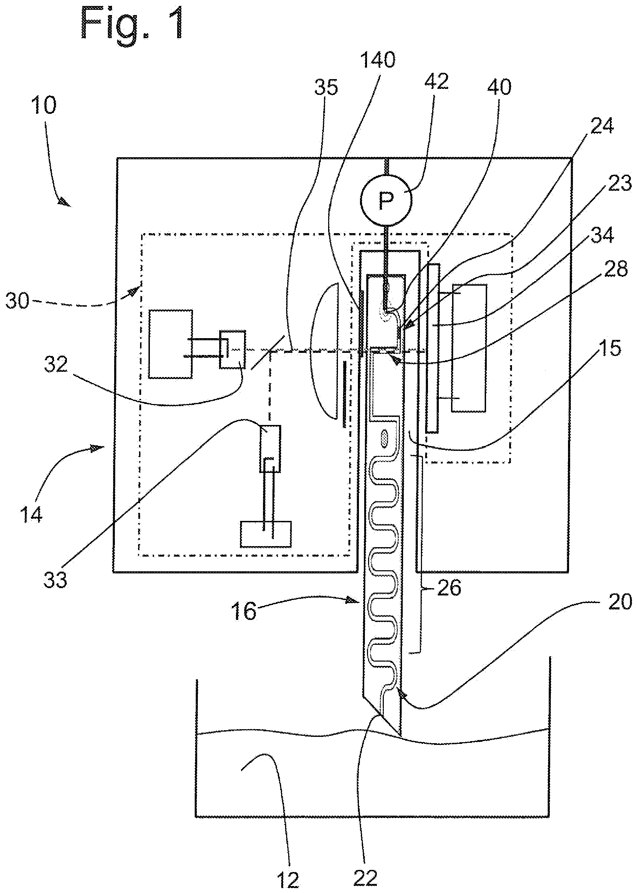

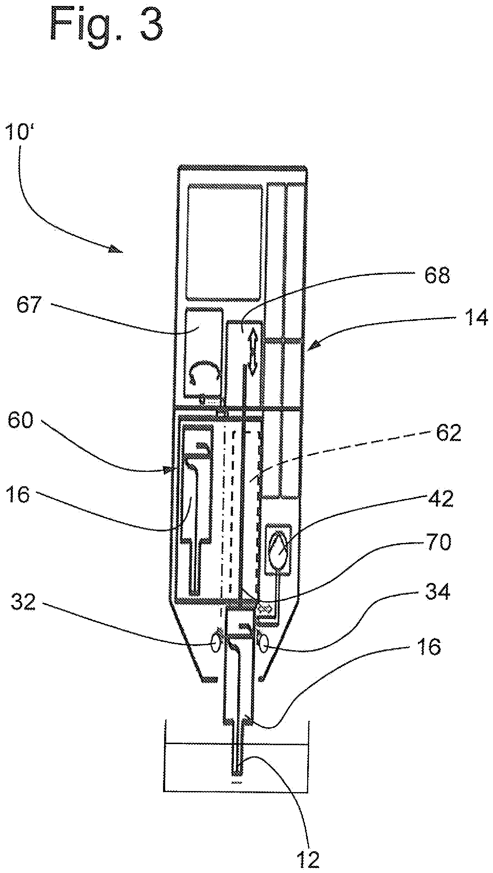

In den Figuren 1 und 3 ist jeweils eine mobile Wasser-Analyseanordnung 10,10' zur quantitativen Bestimmung eines Analyts in einer Wasserprobe schematisch dargestellt. Vorliegend ist eine fotometrische Analyseanordnung 10,10' dargestellt und beschrieben, mit der beispielsweise Chlor, Phosphat oder Ammonium bestimmt werden kann. Grundsätzlich kann die Analyseanordnung alternativ oder ergänzend auch als elektrochemische Analyseanordnung ausgebildet sein.

Die Analyseanordnung der Figur 1 gliedert sich in eine Basiseinheit 14 und ein austauschbares Einmal-Testelement 16, das vorliegend in die Basiseinheit 14 eingesteckt ist.

Das Testelement 16 wird von einem Testelement-Körper 18 aus Kunststoff gebildet, in den eine Probenleitung 20 nutartig eingelassen ist. Der Testelement-Körper 18 ist auf der Seite der Nutöffnung durch eine nicht dargestellte Kunststoff- oder Aluminium-Folie verschlossen.

Die Probenleitung 20 weist an ihrem in Bezug auf die Basiseinheit 14 distalen Ende eine Einlassöffnung 22 auf, durch die eine Wasserprobe aus einem Wasservorrat 12 angesaugt werden kann. Im distalen Bereich der Probenleitung 20 ist ein getrocknetes Hauptreagenz 24 angeordnet. An das Hauptreagenz 24 schließt sich in Fließrichtung ein mäanderartig verlaufender Mischbereich 26 der Probenleitung 20 an, in der das Hauptreagenz 24 und die angesaugte Wasserprobe homogen miteinander vermischt werden,

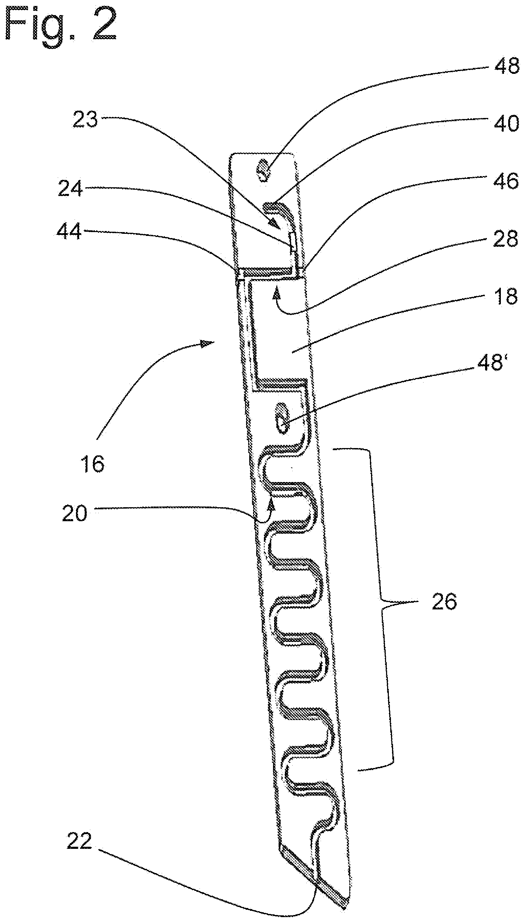

An den Mischbereich 26 schließt sich ein Messabschnitt 28 an, in dem das Analyt quantitativ bestimmt wird. Der Messabschnitt 28 ist vorliegend ein Fotometerabschnitt, der die Messstrecke für ein entsprechendes Fotometer-Messgerät 30 der Basiseinheit 14 bildet. An beiden Enden des Messabschnittes 28 ist jeweils ein für den Messstrahl transparentes Fenster 44,46 vorgesehen, wie im Figur 2 dargestellt. Der Testelement-Körper 18 kann vollständig aus einem für den Messstrahl 35 transparenten Kunststoff bestehen. Die von dem Messabschnitt 28 gebildete Messstrecke wird von einem linearen Längsabschnitt der Probenleitung 20 gebildet, das heißt, die Messstrecke verläuft entlang der gedachten Längsachse der Probenleitung 20 in dem Messabschnitt 28. Hierdurch lässt sich eine erheblich längere Messstrecke realisieren, als dies der Fall wäre, wenn die Messstrecke in Querrichtung der Probenleitung 20 verlaufen würde.

An dem der Einlassöffnung 22 gegenüberliegenden Ende der Probenleitung 20 ist als Pumpelement eine Pumpöffnung 40 vorgesehen, die bei eingestecktem Testelement 16 mit einer einen Pumpaktuator bildenden basiseinheitseitigen Probenpumpe 42 verbunden ist.

Die Basiseinheit 14 weist als Messgerät 30 ein Transmissions-Fotometer auf, das zwei Lichtquellen 32,33 verschiedener Wellenlänge sowie einen Lichtempfänger 34 aufweist.

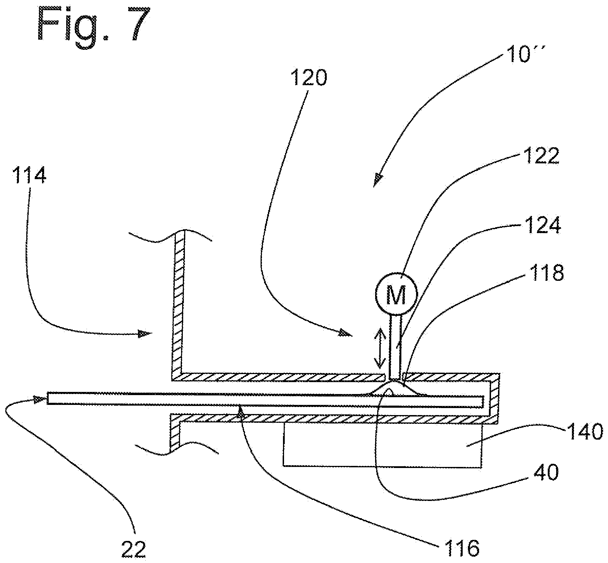

Ferner weist die Basiseinheit 14; 114 ein in der Fig. 7 erkennbares Heiz-Kühlelement 140 in Form eines Peltierelementes zum temperaturgeregelten Heizen beziehungsweise Kühlen des Testelementes 16 auf. Das Heizen beziehungsweise Kühlen ist so geregelt, dass eine konstante Temperatur des Testelementes 16; 116 eingestellt wird. Das Heizen des Testelementes 16; 116 auf beispielsweise 40°C kann dazu dienen, die Reaktion zwischen dem Analyt in der Wasserprobe und dem Hauptreagenz 24 erheblich zu beschleunigen beziehungsweise die Reaktion zu stabilisieren. Das Kühlen des Testelementes 16 beziehungsweise der Wasserprobe in der Probenleitung 20 kann insbesondere die Ausgasung, also die Bildung von die Fotometrie behindernden Gasblasen, verhindern.

Das Testelement 16 weist zwei Positionierelemente 48,48' auf, die als durchgehende Öffnungen ausgebildet sind. Die Positionierelemente 48,48' wirken jeweils mit einem entsprechenden vorgespannten Rastelement der Basiseinheit 14 derart zusammen, dass das Testelement 16 exakt und reproduzierbar in der Basiseinheit 16 positioniert ist. Hierdurch wird sichergestellt, dass der von den Lichtquellen 32,33 generierte Messstrahl 35 exakt fluchtet mit dem fotometrischen Messabschnitt 28. Die Testelement-Aufnahme der Basiseinheit 14 wird von einem Schacht 15 gebildet, in dem das Testelement 16 weitgehend spielfrei eingeschoben ist.

In der Figur 3 ist ein zweites Ausführungsbeispiel einer mobilen Wasser-Analyseanordnung 10' dargestellt, die eine austauschbare Kassette 60 aufweist, die als Trommel mit 15 Fächern 62 für jeweils ein Testelement 16 ausgebildet ist. Der Kunststoff-Trommelkörper 64 ist axial jeweils durch kreisförmige Versiegelungs- Folien 66 derart abgeschlossen, dass die Fächer 62 gas- und flüssigkeitsdicht versiegelt sind.

Wie in der Figur 3 dargestellt ist, ist die austauschbare Kassette 60 in eine entsprechende Kassetten-Aufnahme der Basiseinheit 14' eingesetzt. Die Basiseinheit 14' weist einen Kassetten-Drehantrieb 67 und einen Testelement-Schieberantrieb 68 mit einem Testelement-Schieber 70 auf. Der Schieber 70 kann automatisch ein Testelement 16 aus einer Kammer 62 in die in der Figur 3 dargestellte Messposition schieben.

Sobald die Messung beendet ist, schiebt der Schieber 70 das Testelement 16 aus der Messposition und wirft dieses aus der Basiseinheit 14' aus. Anschließend wird der Schieber 70 vollständig aus der Kassette 60 herausgezogen, woraufhin der Drehantrieb 67 die Kassette 60 um einen Kammerwinkel weiterdreht, so dass die nächste ein Testelement 16 aufweisende Kammer 62 mit dem Schieber 70 fluchtet. Sobald der Benutzer einen Messwunsch signalisiert, schiebt der Schieber 70 das Testelement 16 aus der Kammer 62 in die Messposition, und die Messung kann beginnen.

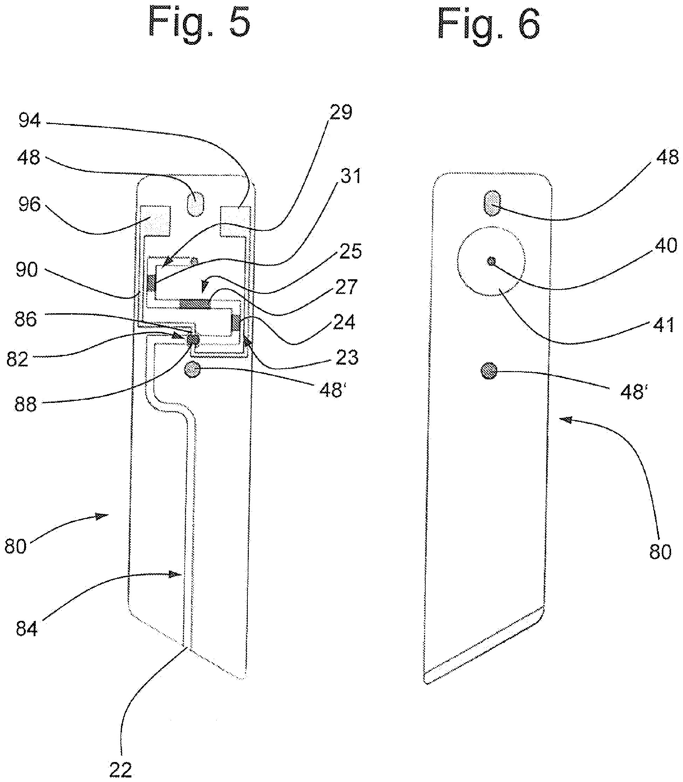

In den Figuren 5 und 6 sind die Vorderseite und die Rückseite eines nicht erfindungsgemässen Testelementes 80 dargestellt. Das Testelement 80 ist ein elektrochemisches Testelement, das einen elektrochemischen Messabschnitt 82 im Verlauf der Probenleitung 84 aufweist. An dem Messabschnitt 82 sind zwei einander gegenüberliegende Elektroden 86,88 vorgesehen, die über Leiterbahnen 90,92 mit Kontakten 94,96 verbunden sind. Die Kontakte 94,96 liegen entsprechenden Kontakten der Basiseinheit gegenüber, wobei letztere mit dem elektrochemischen Messgerät der Basiseinheit verbunden sind,

Auf der in der Figur 6 dargestellten Testelement-Rückseite ist die Pumpöffnung 40 dargestellt. Zur Herstellung einer weitgehend vakuumdichten Anbindung der Probenleitung 84 mit dem als Pumpe ausgebildeten Pumpaktuator 42 ist ein ringförmiges Dichtelement 41 vorgesehen, das die Pumpöffnung 40 umgibt.

In der Figur 7 ist in Seitenansicht schematisch und nur teilweise ein weiteres Ausführungsbeispiel einer mobilen Wasser-Analyseanordnung 10" dargestellt. Die Wasser-Analyseanordnung 10" weist ein Einmal-Testelement 116 auf, das über der Pumpöffnung 40 eine konvexe blasenförmige Pumpenmembrane 118 aufweist. Die ein Pumpelement bildende Pumpenmembrane 118 schließt ein Volumen ein, das größer ist als das gesamte Volumen der Probenleitung 84.

Die Basiseinheit 114 weist als Pumpenaktuator 120 einen Antriebsmotor 122 und einen hiervon angetriebenen Pumpenstößel 124 auf, der die Pumpenmembrane 118 des eingesteckten Einmal-Testelements 116 fein dosierend betätigt. Durch entsprechende Deformation der Pumpenmembrane durch den Pumpenstößel 124 kann die Wasserprobe über die gesamte Länge der Probenleitung beliebig oft vorwärts und rückwärts gefördert werden.

Zur Bestimmung eines Analyts in einer Wasserprobe wird zunächst ein Testelement 16 in die Testelement-Aufnahme 15 der Basiseinheit 14 eingesteckt. Hierdurch wird gegebenenfalls die Basiseinheit 14 eingeschaltet. Daraufhin wird die Einlassöffnung 22 des Testelementes 16 manuell in den zu untersuchenden Wasservorrat 12 eingetaucht, und durch die Pumpe 42 eine Wasserprobe durch Vorwärtsfördern in der Probenleitung 20 bis zu dem Messabschnitt 28 gefördert. In dem Messabschnitt 28 wird mit Hilfe des Messgerätes 30 der Probenblindwert der Wasserprobe bestimmt.

Sobald die Probenblindwert-Bestimmung abgeschlossen ist, wird die Wasserprobe aus dem Messabschnitt 28 vorwärts in den Reagenzabschnitt 23 gefördert. In dem Reagenzabschnitt 23 stößt die Wasserprobe auf das Hauptreagenz 24 und vermischt sich dort mit dem Hauptreagenz 24. Das Hauptreagenz 24 geht mit dem nachzuweisenden Analyt in der Wasserprobe eine Reaktion ein, die die optischen Absorptions-Eigenschaften der Wasserprobe verändert.

Durch Rückwärtsfördern wird die Wasserprobe von dem Reagenzabschnitt 23 wieder zurück in den Messabschnitt 28 gefördert. Dort wird die Wasserprobe durch das Messgerät 30 fotometrisch analysiert. Das Ergebnis dieser Analyse ist ein Brutto-Messwert, Schließlich wird der Blindprobenwert von dem Brutto-Messwert subtrahiert, um auf diese Weise den Netto-Analyt-Messwert, und damit die Konzentration des Analyts in der Wasserprobe zu erhalten.

Mit dem in den Figuren 5 und 6 dargestellten Testelement 80 kann darüber hinaus mit Hilfe des Standard-Additionsverfahrens die Bestimmung des Analyts in der Wasserprobe noch erheblich präzisiert werden. Hierzu wird die Wasserprobe nach der Bestimmung des Analyts in dem Messabschnitt 82 erneut vorwärts bis zu dem ersten Hilfsreagenzabschnitt 25 gefördert, wo sich die Wasserprobe mit dem ersten Hilfsreagenz 27, das ein erstes Analyt-Standard bildet, vermischt.

Anschließend wird die Wasserprobe aus dem ersten Hilfsreagenzabschnitt 25 durch Rückwärtsfördern zurück zu dem Messabschnitt 82 gefördert, wo die Wasserprobe erneut fotometrisch analysiert wird. Schließlich wird die Wasserprobe erneut vorwärts bis in den zweiten Hilfsreagenzabschnitt 29 gefördert, wo sie sich mit dem als ein zweites Analyt-Standard ausgebildeten zweiten Hilfsreagenz 31 vermischt. Von dem zweiten Hilfsreagenzabschnitt 29 wird die Wasserprobe wieder rückwärts zurück zu dem Messabschnitt 82 gefördert, wo sie erneut fotometrisch analysiert wird.

Aus den beiden fotometrischen Analyt-Standard- Analysen kann eine <onzentrations-Absorptions- Kennlinie generiert werden, die eine genaue Bestimmung der Analyt-Konzentration der Wasserprobe aus dem Netto-Analyt- Messwert erlaubt.

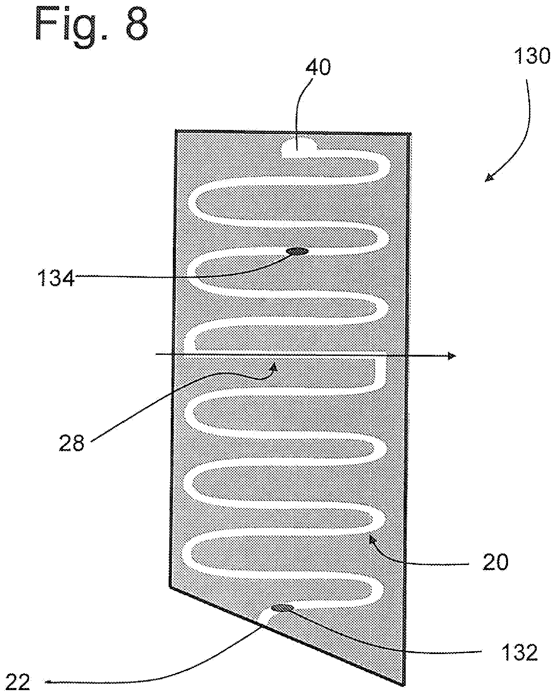

In Figur 8 ist ein weiteres Ausführungsbeispiel eines Testelementes 130 dargestellt, bei dem in unmittelbarer Nähe der Einlassöffnung 22 das Hauptreagenz 132 angeordnet ist. Diese Positionierung des Hauptreagenzes 132 ist insbesondere für ein Chlorreagenz sinnvoll, damit das Analyt Chlor beim Eintritt in die Probenleitung 20 sofort mit dem Chlorreagenz reagiert, bevor das Chlor mit der Wand der Probenleitung 20 reagieren kann. Die Probenleitung 20 beziehungsweise der gesamte Grundkörper des Testelementes 130 besteht in diesem Fall vorzugsweise aus einem in Bezug auf Chlor relativ reaktionsträgen Material, beispielsweise aus Polystyrol.

Zwischen dem Messabschnitt 28 und der Pumpöffnung 40 ist ein Hilfsreagenz 134 angeordnet. Das Hilfsreagenz 134 kann ein separates Reagenz sein, das zum Nachweis eines zweiten Analyts in der Wasserprobe dient. Das Hilfsreagenz 134 kann, aufbauend auf der bzw. nach der Reaktion des Hauptreagenzes 132 mit der Wasserprobe eine mehrstufige Reaktion bewirken. Das Hilfsreagenz 134 kann auch ein Analyt-Standard sein, mit dessen Hilfe eine Standardaddition durchgeführt werden kann.

Das Hilfsreagenz 134 kann auch ein Neutralisationsreagenz sein. Nach der Bestimmung des Analyts in dem Messabschnitt 28 wird die Wasserprobe zu dem Hilfsreagenz 134 gepumpt, das mit dem Hauptreagenz 132 derart reagiert, dass das Hauptreagenz neutralisiert wird, und beispielsweise im Hausmüll entsorgt werden kann.

Das Hilfsreagenz 134 kann auch gelierend und/oder verfärbend in der Wasserprobe wirken, und nach der Durchführung der quantitativen Bestimmung des Analyts in mit der Wasserprobe zusammengeführt werden. Durch die Verfärbung wird deutlich sichtbar gemacht, dass das Testelement 130 bereits benutzt wurde. Durch die Gelierung wird die Wasserprobe in der Probenleitung 20 fixiert, und kann nicht mehr auslaufen.

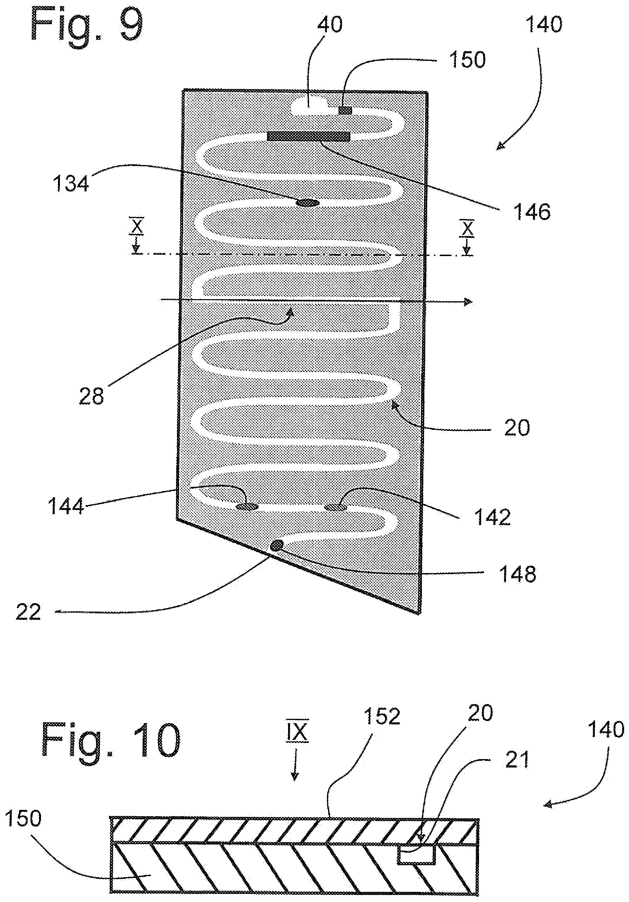

In Figur 9 ist eine weitere Ausführungsform eines Testelementes 140 dargestellt. Hierbei das Hauptreagenz 142 und das Hilfsreagenz 144 relativ dicht hintereinander zwischen der Einlassöffnung 22 und dem Messabschnitt 28 angeordnet. Das Hilfsreagenz 144 kann beispielsweise ein Aktivator sein, der das Hauptreagenz 142 aktiviert, sobald es mit dem Hilfsreagenz 144 in der Wasserprobe vermischt wird. Sowohl das Hauptreagenz als auch das Hilfsreagenz können alternativ jedoch auch zwischen dem Messabschnitt 28 und der Pumpöffnung 40 angeordnet sein.

Zwischen dem Messabschnitt 28 und der Pumpöffnung 40 ist in der Probenleitung 20 ein Absorptionskörper 146 angeordnet, das aus einem Vlieskörper besteht. Unmittelbar angrenzend an die Einlassöffnung 22 ist in der Probenleitung 20 eine Dosierkapillare 148 vorgesehen. Unmittelbar vor der Pumpöffnung 40 kann in der Probenleitung 20 eine hydrophobe Stopperkapillare 150 angeordnet sein, die den Austritt jeder Flüssigkeit aus der Pumpöffnung 40 verhindert. Zwischen der Einlassöffnung 23 und dem Messabschnitt 28, insbesondere nahe der Einlassöffnung 23, ist ein Probenfilter 152 zum Filtern der angesaugten Flüssigkeitsprobe angeordnet.

In Figur 10 ist beispielhaft ein Querschnitt des Einmal-Testelementes 140 der Figur 9 dargestellt. Das Testelement 140 besteht im Wesentlichen aus zwei Teilen, nämlich einem Kunststoff-Bodenteil 150, das spritzgegossen ist und einen separaten Deckelteil 152, das von einer transparenten Kunststoff-Folie gebildet wird, und auf das Bodenteil aufgeschweißt oder aufgeklebt ist. Das Bodenteil 150 weist eine im Querschnitt U-förmige Probenleitungs-Nut 21 auf, die durch das Deckelteil 152 verschlossen ist. Auf diese Weise wird die Probenleitung 20 von dem Bodenteil 150 und dem Deckelteil 152 gebildet.

## Patentansprüche

1. Mobile Wasser-Analyseanordnung (10) zur Bestimmung eines Analyts in einer Wasserprobe, mit einer mobilen Basiseinheit (14) und einem austauschbaren Einmal-Testelement (16), das in die Basiseinheit (14) eingesteckt ist, wobei
das Einmal-Testelement (16) umfasst:
eine Probenleitung (20) mit einer Einlassöffnung (22) zur Aufnahme der Wasserprobe, einem eine Messstrecke bildenden Messabschnitt (28) zur Bestimmung des Analyts und einer Pumpöffnung (40), und
ein Hauptreagenz (24) in der Probenleitung (20),
und die Basiseinheit (14) umfasst:
eine Testelement-Aufnahme (15) zum Halten des eingesteckten Testelementes (16),
ein Messgerät (30), dessen Messstrecke von dem Testelement-Messabschnitt (28) gebildet wird, wobei das Messgerät (30) ein Fotometer (30) mit einer Lichtquelle (32,33) und einem Lichtempfänger (34) zur Erzeugung und zum Empfang eines Messstrahles (35) ist, und
einen Pumpenaktuator, der mit der Pumpöffnung (40) des eingesteckten Testelementes (16) zusammenwirkend verbunden ist, wobei die Messstrecke des Messabschnittes (28) von einem Längsabschnitt der Probenleitung (20) gebildet wird und in Längsrichtung der Probenleitung (20) verläuft.

2. Mobile Wasser-Analyseanordnung (10) nach Anspruch 1, wobei das Einmal-Testelement (16) aus einem Bodenteil (150) und einem separaten Deckelteil (152) besteht, die zwischen sich die Probenleitung (20) bilden.

3. Mobile Wasser-Analyseanordnung (10) nach einem der vorangegangenen Ansprüche, wobei das Testelement (116) eine Pumpenmembrane (118) aufweist, die von dem basiseinheitseitigen Pumpenaktuator (122, 124) betätigt wird.

4. Mobile Wasser-Analyseanordnung (10) nach einem der Ansprüche 1 bis 3 , wobei der Pumpenaktuator ein Teil einer basiseinheitseitigen Probenpumpe (42) ist, und die Probenpumpe (42) mit der Pumpöffnung (40) des eingesteckten Testelementes (16) verbunden ist.

5. Mobile Wasser-Analyseanordnung (10) nach einem der vorangegangenen Ansprüche, wobei in der Probenleitung (20) ein Hilfsreagenz (134) vorgesehen ist.

6. Mobile Wasser-Analyseanordnung (10) nach einem der vorangegangenen Ansprüche, wobei zwischen dem Messabschnitt (28) und der Pumpöffnung (22) ein Absorptionskörper (146) angeordnet ist.

7. Mobile Wasser-Analyseanordnung (10) nach einem der vorangegangenen Ansprüche, wobei in der Probenleitung (20) ein Probenfilter (152) angeordnet ist.

8. Mobile Wasser-Analyseanordnung (10) nach einem der vorangegangenen Ansprüche, wobei die Basiseinheit ein Heiz- und/oder Kühlelement (140) zum Heizen beziehungsweise Kühlen des Testelementes (16) aufweist.

9. Mobile Wasser-Analyseanordnung (10) nach einem der vorangegangenen Ansprüche, wobei in der Probenleitung (20) hydrophobe Stopperkapillare (150) angeordnet ist.

10. Mobile Wasser-Analyseanordnung (10) nach einem der vorangegangenen Ansprüche, wobei in der Probenleitung (20) eine Dosierkapillare (148) angeordnet ist.

11. Verfahren zur Bestimmung eines Analyts in einer Wasserprobe mit einer mobilen Wasser-Analyseanordnung (10) nach einem der Ansprüche 1 bis 10, mit den Verfahrensschritten:
Einstecken eines Testelementes (16) in die Basiseinheit (14),
Eintauchen der Einlassöffnung (22) in das zu untersuchende Wasser,
Einschalten des Pumpenaktuators, wodurch eine Wasserprobe durch die Einlassöffnung (22) in Richtung Messabschnitt (28) gefördert wird,
Isolieren einer definierten Menge der Wasserprobe in der Probenleitung (20),
Mischen der Wasserprobe mit dem Hauptreagenz (24) durch Fördern der Wasserprobe,
Fördern der Wasserprobe bis zu dem Messabschnitt (28),
Analysieren der Wasserprobe in dem Messabschnitt (28) mit Hilfe des Messgerätes (30), und
Entfernen des Testelementes (16) von der Basiseinheit (14).

## Claims

1. A mobile water analysis arrangement (10) for determining an analyte in a water sample, comprising a base unit (14) and a replaceable single-use test element (16) inserted into the base unit (14), wherein
the single-use test element (16) includes:
a sample line (20) with an inlet opening (22) for receiving the water sample, a measuring section (28) forming a measuring path for determining the analyte, and a pump opening (40), and
a main reagent (24) in the sample line (20),
and the base unit (14) includes:
a test element retainer (15) for holding the inserted test element (16),
a measuring apparatus (30), whose measuring path is formed by the test element measuring section (28), wherein the measuring apparatus (30) is a photometer (30) with a light source (32, 33) and a light receiver (34) for generating and receiving a measuring beam (35), and
a pump actuator cooperatively connected with the pump opening (40) of the test element (16) inserted, wherein the measuring path of the measuring section (28) is formed by a longitudinal section of the sample line (20) and extends in the longitudinal direction of the sample line (20).

2. The mobile water analysis arrangement (10) of claim 1, wherein the single-use test element (16) is formed by a bottom part (150) and a separate cover part (152) which form the sample line (20) between them.

3. The mobile water analysis arrangement (10) of one of the preceding claims, wherein the test element (116) comprises a pump membrane (118) operated by the pump actuator (122, 124) on the side of the base unit.

4. The mobile water analysis arrangement (10) of one of claims 1 to 3, wherein the pump actuator is a part of a sample pump (42) on the side of the base unit, and the sample pump (42) is connected with the pump opening (40) of the test element (16) inserted.

5. The mobile water analysis arrangement (10) of one of the preceding claims, wherein an auxiliary reagent (134) is provided in the sample line (20).

6. The mobile water analysis arrangement (10) of one of the preceding claims, wherein an absorption body (146) is arranged between the measuring section (28) and the pump opening (22).

7. The mobile water analysis arrangement (10) of one of the preceding claims, wherein a sample filter (152) is arranged in the sample line (20).

8. The mobile water analysis arrangement (10) of one of the preceding claims, wherein the base unit comprises a heating and/or cooling element (140) for heating or cooling the test element (16).

9. The mobile water analysis arrangement (10) of one of the preceding claims, wherein a hydrophobic stopper capillary (150) is arranged in the sample line (20).

10. The mobile water analysis arrangement (10) of one of the preceding claims, wherein a dosing capillary (148) is arranged in the sample line (20).

11. A method for determining an analyte in a water sample using a mobile water analysis arrangement (10) of one of claims 1 to 10, comprising the following steps:
inserting a test element (16) into the base unit (14),
immersing the inlet opening (22) into the water to be tested,
activating the pump actuator, whereby a water sample is pumped through the inlet opening (22) towards the measuring section (28),
isolating a defined volume of the water sample in the sample line (20),
mixing the water sample with the main reagent (24) by conveying the water sample,
feeding the water sample to the measuring section (28),
analyzing the water sample in the measuring section (28) with the use of the measuring apparatus (30), and
removing the test element (16) from the base unit (14).

## Revendications

1. Dispositif mobile d'analyse d'eau (10) pour la détermination d'un analyte dans un échantillon d'eau, avec une unité de base (14) mobile et un élément de test à usage unique (16) échangeable qui est inséré dans ladite unité de base (14), dans lequel
ledit élément de test à usage unique (16) comprend:
un conduit d'échantillon (20) avec une ouverture d'entrée (22) pour admettre ledit échantillon d'eau, une section de mesurage (28), formant une distance de mesurage, pour la détermination dudit analyte, et une ouverture de pompage (40), et
un réactif principal (24) dans ledit conduit d'échantillon (20).
et ladite unité de base (14) comprend:
un logement (15) pour ledit élément de test pour supporter ledit élément de test (16) inséré,
un appareil de mesurage (30) dont la distance de mesurage est formée par ladite section de mesurage (28) dudit élément de test, ledit appareil de mesurage (30) étant un photomètre (30) avec une source de lumière (32, 33) et un récepteur de lumière (34) pour générer et recevoir un rayon de mesurage (35), et
un actionneur de pompe relié coopérativement avec ladite ouverture de pompage (40) dudit élément de test (16) inséré, ladite distance de mesurage de ladite section de mesurage (28) étant formée par une section longitudinale du conduit d'échantillon (20) et s'étendant dans la direction longitudinale du conduit d'échantillon (20).

2. Dispositif mobile d'analyse d'eau (10) selon la revendication 1, dans lequel ledit élément de test à usage unique (16) est formé par une partie de fond (150) et une partie de couvercle (152) séparée, qui forment, entre eux, ledit conduit d'échantillon (20).

3. Dispositif mobile d'analyse d'eau (10) selon l'une quelconque des revendications précédentes, dans lequel ledit élément d'échantillon (116) comprend une membrane de pompe (118) actionnée par ledit actionneur de pompe (122, 124), côté unité de base.

4. Dispositif mobile d'analyse d'eau (10) selon l'une des revendications 1 à 3, dans lequel ledit actionneur de pompe fait partie d'une pompe d'échantillon (42), côté unité de base, et ladite pompe d'échantillon (42) est reliée à ladite ouverture de pompage (40) dudit élément de test (16) inséré.

5. Dispositif mobile d'analyse d'eau (10) selon l'une quelconque des revendications précédentes, dans lequel un réactif auxiliaire est disposé dans ledit conduit d'échantillon (20).

6. Dispositif mobile d'analyse d'eau (10) selon l'une quelconque des revendications précédentes, dans lequel un corps d'absorption (146) est disposé entre ladite section de mesurage (28) et ladite ouverture de pompage (22).

7. Dispositif mobile d'analyse d'eau (10) selon l'une quelconque des revendications précédentes, dans lequel un filtre d'échantillon (152) est disposé dans ledit conduit d'échantillon (20).

8. Dispositif mobile d'analyse d'eau (10) selon l'une quelconque des revendications précédentes, dans lequel ladite unité de base comprend un élément de chauffage et/ou de refroidissement (140) pour chauffer ou refroidir ledit élément de test (16).

9. Dispositif mobile d'analyse d'eau (10) selon l'une quelconque des revendications précédentes, dans lequel un capillaire de stoppage (150) hydrophobe est disposé dans ledit conduit d'échantillon (20).

10. Dispositif mobile d'analyse d'eau (10) selon l'une quelconque des revendications précédentes, dans lequel un capillaire de dosage (148) est disposé dans ledit conduit d'échantillon (20).

11. Procédé de détermination d'un analyte dans un échantillon d'eau à l'aide d'un dispositif mobile d'analyse d'eau (10) selon l'une des revendications 1 à 10, avec les étapes suivantes:
insérer un élément de test (16) dans ladite unité de base (14),
immerger ladite ouverture d'entrée (22) dans l'eau à analyser,
activer ledit actionneur de pompe, provoquant le refoulement d'un échantillon d'eau à travers l'ouverture d'entrée (22) vers ladite section de mesurage (28),
isoler une quantité définie dudit échantillon d'eau dans ledit conduit d'échantillon (20),
mélanger ledit échantillon d'eau avec ledit réactif principal (24) par refoulement dudit échantillon d'eau,
refouler ledit échantillon d'eau jusqu'à ladite section de mesurage (28),
analyser ledit échantillon d'eau dans ladite section de mesurage (28) à l'aide dudit appareil de mesurage (30), et
enlever ledit élément de test (16) de ladite unité de base (14).
